# EUROPÄISCHE PATENTANMELDUNG

(11) **EP 0 603 615 A2**
(43) Veröffentlichungstag der Anmeldung: **29.06.1994**
(21) Anmeldenummer: 93119575.4
(22) Anmeldetag: 04.12.1993
(51) Int. Cl.: C12N 7/02

(54) **Verfahren zur Reinigung und Anreicherung von Rubella-Virus**

(30) Priorität: 22.12.1992 DE 4243491
(71) Anmelder: BEHRINGWERKE Aktiengesellschaft, 35001 Marburg (DE)
(72) Erfinder: Bernhardt, Dieter, D-35091 Cölbe (DE); Giesendorf, Bernhard, D-24041 Marburg (DE)

(57) **Zusammenfassung**

Die Erfindung betrifft ein Verfahren zur Reinigung und Anreicherung von Rubella-Virus für diagnostische und therapeutische Zwecke durch reversible Bindung an natürliche oder synthetische Partikel, die Rezeptoren für das Virus tragen.

## Beschreibung

Die Erfindung betrifft ein Verfahren zur Reinigung und Anreicherung von Rubella-Virus für diagnostische und therapeutische Zwecke durch reversible Bindung an natürliche oder synthetische Partikel, die Rezeptoren für das Virus tragen.

Zur Isolierung von z. B. Rubella-Antigen aus dem Überstand infizierter animaler Zellen sind bereits Verfahren bekannt, wie z. B. die Isolierung durch Ultrazentrifugation, die entweder als Pelletierung oder als differentielle Zentrifugation mittels eines Dichtegradienten (A. Paris-Hamelin et al., J. Virol. Meth. 10, 1985, 355 - 361) erfolgt. Weitere Verfahren verwenden die Ultrafiltration über Filterflächen mit unterschiedlichen Abscheidungseigenschaften oder Präzipitationsverfahren wie z. B. die mit Polyethylenglycol (PEG) (D.S. Bowden et al., J. gen. Virol. 65 (1984), 933 - 943).

Bekannt ist auch die Anreicherung und Reinigung von Influenzavirus mittels Erythrozyten (B. Giesendorf et al., Virus Research 1, 1984, 655 - 667). Die Abspaltung erfolgt dabei enzymatisch mit Hilfe von Neuraminidase.

Die bekannten Lösungen zur Reinigung von Rubella-Antigen haben jedoch u. a. den Nachteil, daß das so gewonnene Virusantigen mehr oder weniger stark mit zellulären Proteinen und/oder anderen zellulären Bestandteilen verunreinigt sein kann.

Darüberhinaus treten oft als Verunreinigung Komponenten des für die Zellkultivierung verwendeten foetalen Kälberserums (FKS) auf.

Aufgabe der Erfindung war es daher, ein einfaches Verfahren zu finden, bei dem die Ausbeute verbessert und die Verunreinigungen minimiert werden.

Überraschenderweise wurde gefunden, daß humane oder animale Erythrozyten (die sich in ihrer Dichte und Größe deutlich von Viren unterscheiden) unter bestimmten Bedingungen auch Rubella-Virus reversibel binden, der entstehende Komplex aus Zelle und Virus dann über geeignete Verfahren leicht aus dem Milieu, in dem das Virus vermehrt wurde, abgetrennt werden kann, und unter den erfindungsgemäßen Bedingungen dann das Virus von den Erythrozyten abgespalten und gegebenenfalls weiter angereichert werden kann.

Das erfindungsgemäße Verfahren kann auch zur Reinigung und Anreicherung von solchen Virusbestandteilen verwendet werden, die unter den erfindungsgemäßen Bedingungen reversibel an Erythrozyten binden.

Der Begriff Virus im Sinne dieser Erfindung schließt auch diese Virusbestandteile ein.

Gegenstand der Erfindung ist daher ein Verfahren zur Reinigung und Anreicherung von Rubella-Virus und Viruspartikeln durch reversible Bindung an natürliche oder synthetische Partikel, die Rezeptoren für dieses Virus tragen, wobei die Viruspartikel aus der Suspension isoliert, das Virus von den Partikeln gelöst und anschließend isoliert wird, wobei die Bindung des Virus in Gegenwart von zweiwertigen Ionen, die Freisetzung in Gegenwart eines Chelatbildners für zweiwertige Ionen erfolgt.

Bevorzugt ist dabei ein solches Verfahren, bei dem die zweiwertigen Ionen Mg²⁺ und/oder Ca²⁺ sind.

Bevorzugt ist auch ein solches Verfahren, bei dem der Chelatbildner EDTA ist.

Vorteilhaft ist ein solches Verfahren, bei dem der Partikel ein Erythrozyt ist.

Zur Reinigung und Konzentrierung wird dabei z. B. der virushaltige Überstand infizierter Zellen auf einen pH von 4 - 10 eingestellt, bevorzugterweise 6 - 8, besonders bevorzugterweise etwa 7. Geeignete Methoden dazu sind dem Fachmann bekannt. Die Temperatur beträgt dabei 0 °C bis 30 °C, bevorzugterweise 0 - 10 °C, ganz bevorzugterweise etwa 4 °C.

Die Salzkonzentration der Lösung, die die Viren enthält, wird dabei so eingestellt, daß sie zur Zeit der Bindung der Viren an die Erythrozyten in Bezug auf Mg²⁺ 4 x 10⁻⁴ bis 0,4, bevorzugterweise 10⁻³ bis 10⁻² besonders bevorzugterweise 3-5 x 10 ⁻³ mol/l und in Bezug auf CA²⁺ 3 x 10⁻⁴ bis 0,4, bevorzugterweise 10⁻³ bis 10⁻² besonders bevorzugterweise 2-4 x 10 ⁻³ mol/l enthält. Bevorzugterweise werden als Verbindungen Chloride eingesetzt.

Das erfindungsgemäße Verfahren kann mit jeweils einer Ionenart durchgeführt werden.

Bevorzugterweise werden beide Ionen eingesetzt, ganz bevorzugterweise in etwa gleichen molaren Konzentrationen.

Zu der oben beschriebenen Lösung werden Erythrozyten zugegeben, welche Rubellavirus binden können. Vorteilhafterweise verwendet man Erythrozyten vom Menschen, Meerschwein, Taube, Hammel, Schaf, Kaninchen oder Pferd.

Die Konzentration der Erythrozyten beträgt dabei 0,04 - 4 Vol %, bevorzugterweise etwa 0.4 Vol %.

Die Inkubation erfolgt für 1 min - 48 h, bevorzugterweise 20 min - 4 h, ganz bevorzugterweise etwa 1 h.

Vorteilhaft ist auch ein Verfahren, bei dem aus Gründen der Arbeitsökonomie "über Nacht" inkubiert wird.

Der Komplex aus Virus und Zelle wird durch geeignete Methoden abgetrennt. Hier sind dem Fachmann an sich bekannte Zentrifugations- und Filtrationsverfahren besonders vorteilhaft.

Für die Abspaltung des Virus wird ein Komplexbildner zugegeben, der zweiwertige Kationen komplexiert. Solche Komplexbildner sind dem Fachmann an sich bekannt, bevorzugterweise werden, neben EDTA, biologisch abbaubare Komplexbildner verwendet, wie sie z. B. aus der EP 0 488 168 bekannt sind. Die zur Abspaltung nötige Konzentration des Komplexbildners beträgt vorteilhafterweise etwa 3 x 10⁻² bis 3x10⁻³ mol/l und ganz besonders vorteilhafterweise etwa 7 x 10⁻³ mol/l. Die Abspaltung und Elution erfolgt vorteilhafterweise bei einem pH von 6 - 12, besonders vorteilhafterweise bei einem pH von etwa 9.

Da Erythrozyten und Virus bzgl. Größe und Dichte sehr unterschiedlich sind, können sie anschließend leicht durch geeignete, dem Fachmann an sich bekannte Methoden voneinander getrennt werden, vorteilhafterweise geschieht diese Trennung durch niedertourige Zentrifugation, wobei die Erythrozyten sedimentieren und das Virus im überstand bleibt. Die optimalen Zentrifugationsbedingungen können gegebenenfalls leicht experimentell bestimmt werden.

Vorteilhaft ist auch der Einsatz des Verfahrens in einem Säulen-Verfahren, das die folgenden Schritte einschließt:
a) Herstellen einer Säule mit geeigneten Erythrozyten,
b) Zugabe der virushaltigen Lösung unter Berücksichtigung der erforderlichen Ionenbedingungen, wobei die Bindung an die Erythrozyten erfolgt,
c) Waschen der Säule und anschließend
d) Elution des Virus bzw. der Virusbestandteile durch Zugabe des Elutionspuffers.

Nach der Offenbarung des erfindungsgemäßen Verfahrens ist es für den Fachmann leicht, ein solches Säulenverfahren zu optimieren.

Von den genannten Verfahrensschritten sind Variationen denkbar, die auf das vorliegende Erfindungsprinzip zurückgeführt werden können. So kann z. B. der zelluläre Virusrezeptor nach Isolierung, z. B. aus den Erythrozyten, verwendet werden. Der Virusrezeptor kann hierbei an sedimentierbare Kunststoffpartikel oder magnetisierbare Träger gekoppelt sein.

Ein solcher Virusrezeptor kann auch auf alternativem Weg, z. B. gentechnologisch, hergestellt werden.

Der Vorteil des vorliegenden Verfahrens ist, daß das Virus mit einfachen Mitteln ankonzentriert und gleichzeitig von unerwünschten Kontaminanten befreit wird.

Das folgende Beispiel soll die Erfindung erläutern, sie aber nicht einschränken.

### Beispiel:

### Erforderliche Materialien

- 10 l Röteln-Rohvirussuspension
- fixierte Hammelerythrozyten
- 2 n HCl
- 2 l PBS, pH 7,0 mit
   0,1 g/l MgCl₂ x 6 H₂O und
   0,132 g/l CaCl₂ x 2 H₂0
- 0,2 l PBS, pH 7,5
- 0,5 g EDTA

### Adsorption

10 l Röteln-Rohvirussuspension wurden auf 4 °C abgekühlt und mit 2 N HCl auf pH 7 eingestellt. Es wurden 160 ml Erythrozytensuspension (25 Vol %) zugegeben; dies entspricht einer Endkonzentration von 0,4 Vol %. Der Ansatz wurde bei 4 °C für 1 - 16 h mit einem Rührwerk gerührt. Die Suspension wurde in 1 l Zentrifugenbechern in einer Cryofuge 8000 (Heraeus, Deutschland) bei 4 °C und 2700 rpm 10 min zentrifugiert.

Nach der Zentrifugation wurde das Erythrozytenpellet in 1000 ml PBS, pH 7.0 resuspendiert. Es wurde eine zweite Zentrifugation unter o. g. Bedingungen durchgeführt, der Überstand wurde dekantiert.

### Herstellung des Elutionspuffers

Zu PBS pH 7,5 ohne (Mg²⁺/Ca²⁺) wurden 0,25 % EDTA (C₁₀H₁₄N₂O₈Na₂ . 2H₂O; SERVA, Best.-Nr. 11280) zugesetzt und vollständig gelöst. Anschließend wurde mit 2 N NaOH auf pH 9 titriert und die Lösung im Kühlraum auf 4 °C abgekühlt.

Der Elutionspuffer wird möglichst jeweils frisch hergestellt.

Das nach o. g. Verfahren gewonnene Erythrozytenpellet wurde in 1/100 des Volumens der verwendeten Rohvirussuspension (10 l -> 100 ml) Elutionspuffer sorgfältig suspendiert und bei 4 ° C 15 min leicht gerührt.

Die Suspension wurde in einer Cryofuge 8000 bei 4 °C, 3000 rpm 10 min zentrifugiert. Der überstand enthielt den größten Teil des eingesetzten Virus und wurde als "Eluat 1" bezeichnet. Zur Klärung von restlichen Erythrozyten wurde Eluat 1 nochmals zentrifugiert: 4 °C, 3000 rpm, 10 min.

Unter identischen Bedingungen wurde das Erythrozytenpellet nochmals eluiert; hierbei entstand "Eluat 2".

Die Eluate können gepoolt oder getrennt weiterverarbeitet werden.

Zur Kontrolle der Reinigung wurde u. a. eine Immunoblot-Auswertung eingesetzt. Dabei erfolgte die Proteinauftrennung in einem reduzierenden SDS-Polyacrylamidgel (Laemmli, U.K., Nature 227 (1970), S. 680 - 685), Transfer der Proteine auf Nitrozellulose (Bowen, B. et al., Nucleic Acids Res. 8 (1980), S. 1 - 20) und Nachweis der Proteinbanden durch folgendes, dem Fachmann an sich bekanntes Detektionssystem, wobei Röteln-Immunglobulin, biotinyliertes anti-Human-Immmunglobulin und Peroxydasemarkiertes Streptavidin eingesetzt wurden.

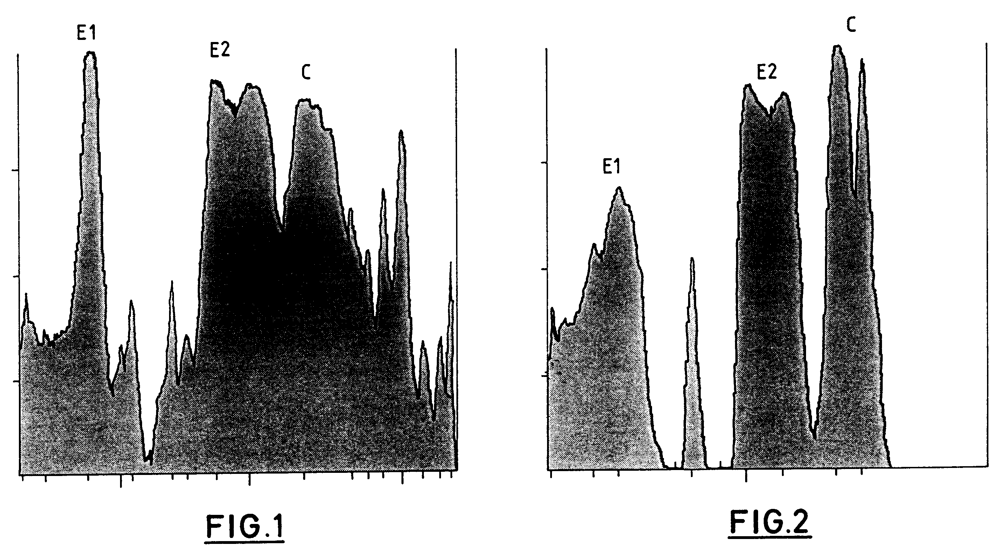

Die Auswertung eines solchen Immunblots mittels Scanner ist in den Figuren 1 und 2 dargestellt, wobei mit E1, E2 und C Strukturproteine des Rubella-Virus gekennzeichnet sind. PEGAg (Fig. 1) ist nach dem Stand der Technik (Bowden et al. (1984)) aufgearbeitetes Material, EryAg (Fig. 2) ist gemäß dem erfindungsgemäßen Verfahren gereinigtes Material.

Zum Scannen wurde ein 2D-1D Videodensitometer (Biotech/Fischer-Software) eingesetzt (peak height: 5, integration resolution: 100 %, peak width: 2, graph length: 15).

### Beschreibung der Figuren

- Figur 1:: Immunoblot-Auswertung eines Rubella-Antigens, das durch PEG-Präzipitation erhalten wurde.
- Figur 2:: Immunoblot-Auswertung eines Rubella-Antigens, das durch das erfindungsgemäße Verfahren isoliert wurde.
- E1, E2, C:: Strukturproteine des Rubella-Virus

## Patentansprüche

1. Verfahren zur Reinigung und Anreicherung von Rubella-Virus durch reversible Bindung an natürliche oder synthetische Partikel, die Rezeptoren für dieses Virus tragen, wobei das Virus aus einer Suspension isoliert, von den Partikeln gelöst und anschließend isoliert wird dadurch gekennzeichnet, daß die Bindung des Virus in Gegenwart von zweiwertigen Ionen, die Freisetzung in Gegenwart eines Chelatbildners für zweiwertige Ionen erfolgt.

2. Verfahren nach Anspruch 1 dadurch gekennzeichnet, daß die zweiwertigen Ionen Mg²⁺ und/oder Ca²⁺ sind.

3. Verfahren nach Anspruch 1 oder 2 dadurch gekennzeichnet, daß der Chelatbildner EDTA ist.

4. Verfahren nach Anspruch 1 dadurch gekennzeichnet, daß der Partikel ein Erythrozyt ist.

5. Verfahren nach Anspruch 1 dadurch gekennzeichnet, daß der Partikel ein synthetischer Partikel ist, an den isolierter Virusrezeptor gebunden ist.
